# EUROPEAN PATENT SPECIFICATION

(11) **EP 2 793 854 B1**
(45) Date of publication and mention of the grant of the patent: **16.12.2015**
(21) Application number: 12824787.1
(22) Date of filing: 20.12.2012
(51) Int. Cl.: A61K 9/00, A61K 31/00, A61K 47/00

(54) **FORMULATIONS OF FLURBIPROFEN AND DIACEREIN**
FORMULIERUNGEN AUS FLURBIPROFEN UND DIACEREIN
FORMULATIONS DE FLURBIPROFÈNE ET DE DIACÉRÉINE

(30) Priority: 23.12.2011 TR 201112836; 27.12.2011 TR 201113006
(43) Date of publication of application: 29.10.2014
(73) Proprietor: Sanovel Ilac Sanayi Ve Ticaret Anonim Sirketi, 34460 Istanbul (TR)
(72) Inventor: CIFTER, Umit, 34460 Istanbul (TR); TURKYILMAZ, Ali, 34460 Istanbul (TR); PEHLIVAN AKALIN, Nur, 34460 Istanbul (TR); ZENGINER, Sibel, 34460 Istanbul (TR)
(74) Representative: Sevinç, Erkan
(86) International application number: PCT/TR2012/000244
(87) International publication number: WO 2013/095319

(56) References cited:
- GB-A- 2 432 526
- US-A- 4 996 209
- US-A1- 2004 039 366

## Description

### Field of Invention

The present invention relates to novel pharmaceutical formulations of flurbiprofen or a pharmaceutically acceptable salt of flurbiprofen and diacerein or a pharmaceutically acceptable derivative of diacerein, having anti-inflammatory, analgesic, antipyretic, and osteoarthritis-treating activities.

The present invention particularly relates to orally-administered pharmaceutical formulations of flurbiprofen and diacerein, having anti-inflammatory, analgesic, antipyretic, and osteoarthritis-treating activities. The solubility, dissolution rate, and stability of said formulation are improved based on the excipients comprised therein.

### Background of Invention

Flurbiprofen is a propionic acid derivative, also known as NSAID (non-steroidal anti-inflammatory drug), having analgesic and anti-inflammatory activities. Its chemical structure is illustrated with Formula 1 given below.

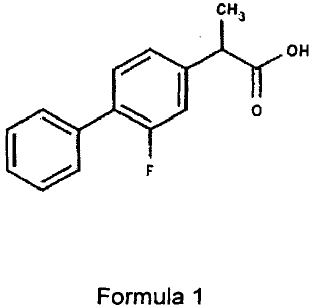

Flurbiprofen is used for alleviating pain in muscle-skeleton system and joint disorders such as ankylosing spondylitis, osteoarthritis, and rheumatoid arthritis, in soft tissue injuries such as sprains and strains, in postoperative cases, and in painful and severe menstruation and migraine. Flurbiprofen is further used as a lozenge in the symptomatic amelioration of sore throats.

Flurbiprofen sodium is used in eye drops for preventing intraoperative miosis, as well as for controlling the inflammation of the eye's anterior layer following surgery. Flurbiprofen is administered via intravenous injection against severe pains in some countries.

The application WO 98/52545 relates to pharmaceutical compositions comprising a formulation of flurbiprofen with a therapeutically effective amount of one or more active ingredients selected from an antihistamine, a cough suppressant, a decongestant, an expectorant, a muscle relaxant, a centrally acting analgesic, a local anesthetic, an antibacterial compound, an antiviral compound, an antibiotic compound, an antifungal compound, minerals and vitamins and/or a burn-masking amount of an agent which has a warming effect on the mucosa of the throat.

The patent US05807568 discloses a topically-administered formulation comprising flurbiprofen as the active agent.

The patent WO9523596 discloses a flurbiprofen solution in a C2-4 alcohol.

Diacerein, having the chemical structure given below with Formula 2, is an interleukin-1 inhibitor having an anthraquinone structure and is used for treating osteoarthritis.

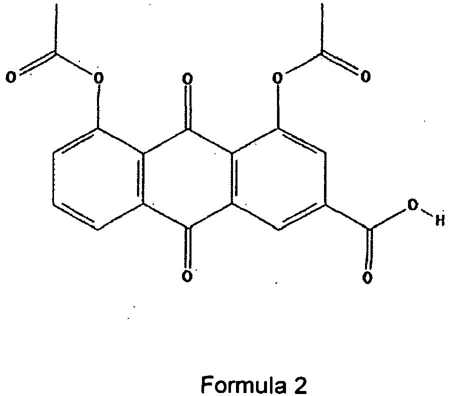

Diacerein specifically provides alleviation of the symptoms of osteoarthritis. There are various patent applications which comprises diacerein formulations.

The application EP0809995 discloses a formulation of diacerein, which is characterized in that diacerein is incorporated into a hydrogel matrix containing at least a ionic polysaccharide gelled by addition of electrolytes.

The application EP0862423 discloses a pharmaceutical composition for oral delivery, including a liquid carrier oil selected from vegetable, animal or mineral oils, a suspending agent, a homogenizing agent, and a surfactant, said composition being characterized by comprising diacerein, rein or one of a pharmaceutically acceptable salt thereof.

The application EP0904061 discloses a pharmaceutical composition for administration by oral, rectal or cutanoues route, having improved bioavailability, characterized by comprising rein or diacerein and sodium laurylsulphate in a weight ratio between 3:1 and 10:1.

No orally-administrable pharmaceutical formulation has been produced until today containing a combination of flurbiprofen and diacerein. Even if some medicaments comprising either of these active agents have been administered concomitantly in practice, this fact requires the patients to carry more than one drugs and causes application-related difficulties.

The use of flurbiprofen and diacerein in treating osteoarthritis, pain, and inflammations may cause a problem especially for those who have gastrointestinal system disorders. Both of these active agents lead to a complaint in the form of burning sensation in the gastrointestinal system. Preventing the systemic side effects of flurbiprofen and diacerein is quite important in terms of patient compliance. Various coating processes have been performed to prevent such side effects. The coatings, however, may cause problems in terms of solubility and thus bioavailability. Improving the solubility and therefore the absorption both provides ease of application and increases the molecule's efficiency.

Another problem in relation to flurbiprofen and diacerein is that these molecules are weakly soluble in water. This, in turn, directly effects the bioavailability, and therefore the solubility and dissolution rate have to be increased.

Another problem in relation to both of these active agents is stability, which emerges under the influence of ambient and physical conditions, as is the case with many other active agents. These active agents are influenced both from temperature, air, and humidity conditions, and from the solvents used while they are formulated. When they are exposed to air and humidity, said active agents degrade structurally and develop chemical behavioral changes. The stability of the products developed is not at a desired level and the shelf life thereof is shortened. In addition, these active agents are reactive against the excipients employed in developing the formulations containing the same. This, in turn, causes impurities to occur in the formulations and leads to the inclusion of undesired components into the formulations. It is of critical importance in terms of the formulation to use those excipients and methods which do not lead to said problems.

In result, a novelty is required in the art of pharmaceutical formulations having anti-inflammatory, analgesic, antipyretic, and osteoarthritis-treating activities due to the aforesaid drawbacks.

### Object and Brief Description of Invention

The present invention provides a combination formulation of flurbiprofen and diacerein, eliminating all aforesaid problems and bringing additional advantages to the relevant prior art.

Accordingly, the main object of the present invention is to obtain at least one stable formulation with anti-inflammatory, analgesic, antipyretic, and osteoarthritis-treating activities.

Another object of the present invention is to provide a formulation having a desired solubility and dissolution rate, and therefore a desired level of bioavailability, with this formulation comprising flurbiprofen and diacerein produced by means of a hot-melt method.

A further object of the present invention is to eliminate the need for any liquid solvent, including water.

Another object of the present invention is to prevent or minimize the burning sensation occurring in the gastrointestinal system by the excipients and production method used to obtain the formulation.

A further object of the present invention is to obtain a uniform formulation content.

Another object of the present invention is to develop a formulation not leading to flowability-related problems during production.

A pharmaceutical formulation is developed to carry out all objects, referred to above and to emerge from the following detailed description.

In a preferred embodiment according to the present invention, said novelty is realized with flurbiprofen or a pharmaceutically acceptable salt of flurbiprofen, and diacerein or a pharmaceutically acceptable salt of diacerein, and polyvinyl caprolactam-polyvinyl acetate-polyethylene glycol graft copolymer.

In another preferred embodiment according to the present invention, said formulation is obtained by means of a hot-melt method not giving place to any liquid solvent during the granulation phase.

In a preferred embodiment according to the present invention, the proportion of flurbiprofen and diacerein to polyvinyl caprolactam-polyvinyl acetate-polyethylene glycol graft copolymer is in the range of 0.1 to 10, preferably 0.15 to 5, and more preferably 0.2 to 2.

Another preferred embodiment of the present invention comprises at least one or more excipient(s).

According to a preferred embodiment of the present invention, said excipient(s) comprise(s) at least one or a properly-proportioned mixture of diluents, binders, disintegrants, glidants, lubricants, and plasticizers.

According to a preferred embodiment of the present invention, the mean particle size (d₅₀) of the granules obtained by means of the hot-melt method is in the range of 100 - 1500 µm, preferably 150 - 1000 µm, and more preferably 250 - 800 µm.

According to a preferred embodiment, the present invention also comprises at least one or a properly-proportioned mixture of polyvinyl alcohol-polyethylene glycol copolymer, polyoxyethylene-polyoxypropylene block copolymer, stearyl macrogol glyceride, polyethylene glycol, povidone, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol, stearic acid, triacetine, triacetine citrate and tripropionin. Polymers with a low glass transition temperature are preferred in the formulation according to the present invention.

In a preferred embodiment of the present invention, said disintegrant is at least one or a properly-proportioned mixture of polyvinylpyrrolidone and sodium starch glycolate.

In a preferred embodiment of the present invention, said glidant is colloidal silicon dioxide.

In a preferred embodiment of the present invention, said lubricant preferably comprises at least one or a properly-proportioned mixture of polyethylene glycol and magnesium stearate.

In a preferred embodiment of the present invention, said plasticizer comprises preferably at least one or a properly-proportioned mixture of castor oil, glycerin, citrate esters (acetyl tri-n-butyl citrate, acetyl triethyl citrate, tri-n-butyl citrate, triethyl citrate) dibutyl sebacate, triacetine, diethyl phthalate, low molecular weight polyethylene glycols. The use of a plasticizer serves to reduce the glass transition temperature of the polymer and to increase the stability of the active agent used in the formulation.

In a preferred embodiment according to the present invention, said pharmaceutical formulation consisting of
a. 15 to 70% by weight of flurbiprofen or a pharmaceutically acceptable salt thereof,
b. 5 to 70% by weight of diacerein or a pharmaceutically acceptable salt thereof,
c. 5 to 80% by weight of polyvinyl caprolactam-polyvinyl acetate-polyethylene glycol graft copolymer or polyvinyl alcohol-polyethylene glycol copolymer,
d. 0.5 to 25% by weight of croscarmellose sodium,
e. 0.1 to 10% by weight of colloidal silicon dioxide,
f. 0.1 to 10% by weight of magnesium stearate,
g. 0.1 to 10% by weight of plasticizer.

In a preferred embodiment according to the present invention, said pharmaceutical formulation consisting of
a. 15 to 70% by weight of flurbiprofen or a pharmaceutically acceptable salt thereof,
b. 5 to 70% by weight of diacerein or a pharmaceutically acceptable salt thereof,
c. 0.5 to 80% by weight of stearyl macrogol glycerides and polyvinyl caprolactam-polyvinyl acetate-polyethylene glycol graft copolymer
d. 0.5 to 25% by weight of croscarmellose sodium,
e. 0.1 to 10% by weight of colloidal silicon dioxide,
f. 0.1 to 10% by weight of magnesium stearate,
g. 0.1 to 10% by weight of plasticizer.

Another preferred embodiment according to the present invention provides a method for preparing said pharmaceutical formulation, this method comprising the steps of
a. mixing flurbiprofen and diacerein, polyvinyl caprolactam-polyvinyl acetate-polyethylene glycol graft copolymer or polyvinyl alcohol-polyethylene glycol copolymer and plasticizer together, melting this mixture, and passing it through an extruder or sieve,
b. adding first croscarmellose sodium and colloidal silicon dioxide, and then magnesium stearate to the granules obtained and mixing the same,
c. performing a compression step on this powder mixture in a tablet machine, or filling this powder mixture into capsules.

Another preferred embodiment according to the present invention provides a method for preparing said pharmaceutical formulation, this method comprising the steps of
a. mixing flurbiprofen and diacerein, stearyl macrogol glycerides and plasticizers together, melting this mixture, and passing it through a sieve or an extruder,
b. adding first croscarmellose sodium and colloidal silicon dioxide, and then magnesium stearate to the granules obtained and mixing the same,
c. performing a compression step on this powder mixture in a tablet machine, or filling this powder mixture into capsules.

In another preferred embodiment of the present invention, said formulation is in the form of a tablet, capsule, or sachet.

### Detailed Description of Invention

### Example 1: Capsule or tablet

| **Ingredients** | **% amount (mg)** |
|---|---|
| flurbiprofen | 15 - 70% |
| diacerein | 5 - 70% |
| polyvinyl caprolactam-polyvinyl acetate-polyethylene glycol graft copolymer or polyvinyl alcohol-polyethylene glycol copolymer | 5 - 80% |
| croscarmellose sodium | 0.5 - 25% |
| colloidal silicon dioxide | 0.1 - 10% |
| magnesium stearate | 0.1 - 10% |
| plasticizer | 0.1 - 10% |

This formulation is produced as follows. Flurbiprofen and diacerein, plasticizer, and polyvinyl caprolactam-polyvinyl acetate-polyethylene glycol graft copolymer or polyvinyl alcohol-polyethylene glycol copolymer are mixed together, this mixture is melted and passed through an extruder or sieve. Into the granules obtained above, first croscarmellose sodium and colloidal silicon dioxide, and then magnesium stearate are added and the resulting mixture is mixed. A compression step is performed on this powder mixture in a tablet machine, or this powder mixture is filled into capsules. The tablets are coated preferably with a humidity-barrier coating material, such as opadry AMB/ kollicoat IR.

### Example 2:

| **Ingredients** | **% amount (mg)** |
|---|---|
| flurbiprofen | 15 - 70% |
| diacerein | 5 - 70% |
| stearyl macrogol glycerides and polyvinyl caprolactam-polyvinyl acetate-polyethylene glycol graft copolymer or polyvinyl alcohol-polyethylene glycol copolymer | 0.5 - 80% |
| croscarmellose sodium | 0.5 - 25% |
| colloidal silicon dioxide | 0.1 - 10% |
| magnesium stearate | 0.1 - 10% |
| plasticizer | 0.1 - 10% |

This formulation is produced as follows. Flurbiprofen and diacerein, plasticizer and stearyl macrogol glycerides are mixed together, this mixture is melted and passed through an extruder or sieve. Into the granules obtained above, first croscarmellose sodium and colloidal silicon dioxide, and then magnesium stearate are added and the resulting mixture is mixed. A compression step is performed on this powder mixture in a tablet machine, or this powder mixture is filled into capsules. The tablets are coated preferably with a humidity-barrier coating material, such as opadry AMB/ kollicoat IR.

With this invention, a stable formulation is obtained which has a surprisingly high solubility and a high dissolution rate. Said formulation comprises flurbiprofen and diacerein and polyvinyl caprolactam-polyvinyl acetate-polyethylene glycol graft copolymer or additionally at least one or a properly-proportioned mixture of polyvinyl alcohol-polyethylene glycol copolymer, polyoxyethylene-polyoxypropylene block copolymer, stearyl macrogol glyceride, polyethylene glycol, povidone, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol, stearic acid, triacetine, triacetine citrate and tripropionin. The method described above both serves to provide a uniform formulation content, and eliminates the need for any liquid solvent including water. Any flowability-related problems encountered during production are prevented as well. In said formulation, the proportion of flurbiprofen and diacerein to polyvinyl caprolactam-polyvinyl acetate-polyethylene glycol graft copolymer or polyvinyl alcohol-polyethylene glycol copolymer is in the range of 0.1 to 10, preferably 0.15 to 5, and more preferably 0.2 to 2. These ranges allow to achieve the desired dissolution rate and solubility. Polymers with low glass transition temperature and melting point are used in said formulation. On the other hand, using a plasticizer which reduces the glass transition temperature increases the stability of the active agent. The plasticizer used in the hot-melt method drops down the glass transition temperature of the polymers used in hot-melting, and thus allows to formulate the active agent at lower temperatures. In result, the formulation is made more stable. One of the important advantages of the developed formulation is the prevention or minimization of the burning sensation occurring in the gastrointestinal system by means of a matrix formed.

The pharmaceutical compositions according to the present invention may also comprise one or more pharmaceutically acceptable excipient(s). Such pharmaceutically acceptable excipients include, but are not limited to fillers, glidants, lubricants, disintegrants, surface active agents etc. and the mixtures thereof.

The present invention is for use in mammalians and particularly in humans for the prevention or treatment of pain, arthralgia, toothache, myalgia, miosis inhibition, ankylosing spondylitis, osteoarthritis, rheumatoid arthritis and other muscle-skeleton system and joint disorders, soft tissue injuries such as sprains and strains, postoperative pains, painful and severe menstruation, migraine, and sore throat.

In this context, the term formulation may both correspond to a formulation, and to a combined meaning of the formulation and the package or blister in which the formulation is stored.

In this context, the term particle comprises a powder, granule, or a pellet.

It is also possible to use the following additional excipients in this formulation.

Diluents, e.g. at least one or a mixture of lactose, microcrystalline cellulose, starch, mannitol, calcium phosphate anhydrate, calcium phosphate dihydrate, calcium phosphate trihydrate, dibasic calcium phosphate, calcium carbonate, calcium sulfate, carboxymethyl cellulose calcium, powdered cellulose, cellulose acetate, pregelatinized starch, lactose monohydrate, corn starch.

Binders, e.g. at least one or a mixture of polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polyethylene oxide, gelatin, starch, xanthan gum, guar gum, alginate, carrageen, pectin, carbomer, cellulose acetate phthalate, hydroxypropyl starch, hydroxyethyl methyl cellulose, polaxomer, polyethylene glycol (PEG).

Lubricants, e.g. at least one or a mixture of sodium stearyl fumarate, polyethylene glycol, stearic acid, metal stearates, boric acid, sodium chloride benzoate and acetate, sodium or magnesium lauryl sulfate, etc..

Preservatives, e.g. at least one or a mixture of methylparaben and propylparaben and salts thereof (e.g. sodium or potassium salts), sodium benzoate, citric acid, benzoic acid, butylated hydroxytoluene and butylated hydroxyanisole, etc..

Surface active agents, e.g. at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate, etc..

The present invention is hereby disclosed by referring to exemplary embodiments hereinabove. Whilst these exemplary embodiments does not restrict the object of the present invention, it must be assessed under the light of the foregoing detailed description.

## Claims

1. A pharmaceutical formulation, **characterized by** comprising flurbiprofen or a pharmaceutically acceptable salt of flurbiprofen, and diacerein or a pharmaceutically acceptable salt of diacerein, and polyvinyl caprolactam-polyvinyl acetate-polyethylene glycol graft copolymer.

2. The pharmaceutical formulation according to Claim 1, wherein said formulation is obtained by means of a hot-melt method not involving any liquid solvent during the granulation phase.

3. The pharmaceutical formulation according to any of the preceding claims, wherein the proportion of flurbiprofen and diacerein to polyvinyl caprolactam-polyvinyl acetate-polyethylene glycol graft copolymer is in the range of 0.1 to 10, preferably 0.15 to 5, and more preferably 0.2 to 2.

4. The pharmaceutical formulation according to any of the preceding claims, further comprising at least one or more excipient.

5. The pharmaceutical formulation according to any of the preceding claims, wherein said excipient selected from the group consisting of at least one or a mixture of diluents, binders, disintegrants, glidants, lubricants, and plasticizers.

6. The pharmaceutical formulation according to any of the preceding claims, wherein the mean particle size (d₅₀) of the granules obtained by means of the hot-melt method is in the range of 100 - 1500 µm, preferably 150 - 1000 µm, and more preferably 250 - 800 µm.

7. The pharmaceutical formulation according to any of the preceding claims, further comprising at least one polymer wherein said polymer selected from the group consisting of at least one or a mixture of polyvinyl alcohol-polyethylene glycol copolymer, polyoxyethylene-polyoxypropylene block copolymer, stearyl macrogol glyceride, polyethylene glycol, povidone, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol, stearic acid, triacetine, triacetine citrate and tripropionin.

8. The pharmaceutical formulation according to any of the preceding claims, wherein said disintegrant selected from the group consisting of at least one or a mixture of croscarmellose sodium and sodium starch glycolate.

9. The pharmaceutical formulation according to any of the preceding claims, wherein said glidant is colloidal silicon dioxide.

10. The pharmaceutical formulation according to any of the preceding claims, wherein said lubricants selected from the group consisting of at least one or a mixture of polyethylene glycol and magnesium stearate.

11. The pharmaceutical formulation according to any of the preceding claims, wherein said plasticizer selected from the group consisting of at least one or a mixture of castor oil, glycerin, citrate esters (acetyl tri-n-butyl citrate, acetyl triethyl citrate, tri-n-butyl citrate, triethyl citrate) dibutyl sebacate, triacetine and diethyl phthalate.

12. The pharmaceutical formulation according to Claim 7, consisting of
a. 15 to 70% by weight of flurbiprofen or a pharmaceutically acceptable salt thereof,
b. 5 to 70% by weight of diacerein or a pharmaceutically acceptable salt thereof,
c. 5 to 80% by weight of polyvinyl caprolactam-polyvinyl acetate-polyethylene glycol graft copolymer or polyvinyl alcohol-polyethylene glycol copolymer,
d. 0.5 to 25% by weight of croscarmellose sodium,
e. 0.1 to 10% by weight of colloidal silicon dioxide,
f. 0.1 to 10% by weight of magnesium stearate,
g. 0.1 to 10% by weight of plasticizer.

13. The pharmaceutical formulation according to any of the preceding claims, consisting of
a. 15 to 70% by weight of flurbiprofen or a pharmaceutically acceptable salt thereof,
b. 5 to 70% by weight of diacerein or a pharmaceutically acceptable salt thereof,
c. 0.5 to 80% by weight of stearyl macrogol glycerides and polyvinyl caprolactam-polyvinyl acetate-polyethylene glycol graft copolymer
d. 0.5 to 25% by weight of croscarmellose sodium,
e. 0.1 to 10% by weight of colloidal silicon dioxide,
f. 0.1 to 10% by weight of magnesium stearate,
g. 0.1 to 10% by weight of plasticizer.

14. A method for preparing a pharmaceutical formulation according to Claim 12, comprising the steps of
a. mixing flurbiprofen and diacerein, plasticizer and polyvinyl caprolactam-polyvinyl acetate-polyethylene glycol graft copolymer or polyvinyl alcohol-polyethylene glycol copolymer together, melting this mixture and passing it through an extruder or a sieve,
b. adding first croscarmellose sodium and colloidal silicon dioxide, and then magnesium stearate to the granules obtained and mixing the same,
c. performing a compression step on this powder mixture in a tablet machine, or filling this powder mixture into capsules.

15. A method for preparing a pharmaceutical formulation according to any of the preceding claims, comprising the steps of
a. mixing flurbiprofen and diacerein, plasticizer, stearyl macrogol glycerides and polyvinyl caprolactam-polyvinyl acetate-polyethylene glycol graft copolymer or polyvinyl alcohol-polyethylene glycol copolymer together, melting this mixture and passing it through an extruder or a sieve,
b. adding first croscarmellose sodium and colloidal silicon dioxide, and then magnesium stearate to the granules obtained and mixing the same,
c. performing a compression step on this powder mixture in a tablet machine, or filling this powder mixture into capsules.

## Patentansprüche

1. Eine pharmazeutische Formulierung, **dadurch gekennzeichnet, dass** sie umfasst Flurbiprofen oder pharmazeutisch annehmbares Salz von Flurbiprofen und Diacerein oder ein pharmazeutisch annehmbares Salz von Diacerein und Polyvinylcaprolactam-Polyvinylacetat-Polyethylenglycol-Pfropfcopolymer.

2. Die pharmazeutische Formulierung nach Anspruch 1, worin die Formulierung erhalten wird mittels eines Heiß-Schmelzverfahrens, das nicht ein beliebiges flüssiges Lösemittel während der Granulierungsphase einschließt.

3. Die pharmazeutische Formulierung nach irgendeinem der vorstehenden Ansprüche, worin der Anteil Flurbiprofen und Diacerein zu Polyvinylcaprolactam-Polyvinylacetat-Polyethylenglycol-Pfropfcopolymer in einem Bereich von 0.1 bis 10 ist, vorzugsweise 0.15 bis 5, und mehr bevorzugt 0.2 bis 2.

4. Die pharmazeutische Formulierung nach irgendeinem der vorstehenden Ansprüche, weiterhin umfassend mindestens einen oder mehrere Hilfsstoffe.

5. Die pharmazeutische Formulierung nach irgendeinem der vorstehenden Ansprüche, worin der Hilfsstoff ausgewählt ist aus der Gruppe, bestehend aus mindestens einem oder einer Mischung aus Verdünnungsmitteln, Desintegrationshilfsmitteln, Gleitmitteln, Schmierstoffen und Weichmachern.

6. Die pharmazeutische Formulierung nach irgendeinem der vorstehenden Ansprüche, worin die mittlere Teilchengröße (d₅₀) der Granula, erhalten mittels des Heiß-Schmelzverfahrens in einem Bereich von 100 - 1500 µm ist, vorzugsweise 150 - 1000 µm und mehr bevorzugt 250 - 800 µm.

7. Die pharmazeutische Formulierung nach irgendeinem der vorstehenden Ansprüche, weiterhin umfassend mindestens ein Polymer, wobei das Polymer ausgewählt ist aus der Gruppe, bestehend aus mindestens einem oder einer Mischung aus Polyvinylalkohol-Polyethylenglycol-Copolymer, Polyoxyethylen-Polyoxypropylen-Block-Copolymer, Stearyl-Macrogol-Glycerin, Polyethylenglycol, Povidon, kationisches Methacrylat, Copovidon, Methacrylsäure-Copolymer-Derivate, Celullose-Acetat-Phthalat, acetyliertes Monoglycerid, Dibutyltartrat, Diethylphthalat, Dimethylphthalat, Glycerin, Propylenglycol, Stearinsäure, Triacetin, Triacetincitrat und Tripropionin.

8. Die pharmazeutische Formulierung nach irgendeinem der vorstehenden Ansprüche, worin das Desintegrationshilfsmittel ausgewählt ist aus der Gruppe, bestehend aus mindestens einem oder einer Mischung aus Croscarmellose-Natrium und Natrium-Stärkeglycolat.

9. Die pharmazeutische Formulierung nach irgendeinem der vorstehenden Ansprüche, worin das Gleitmittel kolloidales Siliziumdioxid ist.

10. Die pharmazeutische Formulierung nach irgendeinem der vorstehenden Ansprüche, worin die Schmiermittel ausgewählt sind aus der Gruppe, bestehend aus mindestens einem oder einer Mischung aus Polyethylenglycol und Magnesiumstearat.

11. Die pharmazeutische Formulierung nach irgendeinem der vorstehenden Ansprüche, worin der Weichmacher ausgewählt ist aus der Gruppe, bestehend aus mindestens einem oder einer Mischung aus Rizinusöl, Glycerin, Citratestern (Acetyl-tri-n-butylcitrat, Acetyl-Triethylcitrat, Tri-n-butylcitrat, Triethylcitrat), Dibutylsebacat, Triacetin und Diethylphthalat.

12. Die pharmazeutische Formulierung nach Anspruch 7, bestehend aus
a. 15 bis 70 Gew. % Flurbiprofen oder einem pharmazeutisch annehmbaren Salz davon,
b. 5 bis 70 Gew. % Diacerein oder einem pharmazeutisch annehmbaren Salz davon,
c. 5 bis 80 Gew. % Polyvinylcaprolactam-Polyvinylacetat-Polyethylenglycol- Pfropfcopolymer oder Polyvinylalkohol-Poyethylenglycol-Copolymer,
d. 0.5 bis 25 % Gew. % Croscarmellose-Natrium,
e. 0.1 bis 10 Gew. % kolloidales Siliziumdioxid,
f. 0.1 bis 10 Gew. % Magnesiumstearat.

13. Die pharmazeutische Formulierung nach irgendeinem der vorstehenden Ansprüche, bestehend aus
a. 15 bis 70 Gew. % Flurbiprofen oder einem pharmazeutisch annehmbaren Salz davon,
b. 5 bis 70 Gew. % Diacerein oder einem pharmazeutisch annehmbaren Salz davon,
c. 0.5 bis 80 Gew. % Stearyl-Macrogol-Glyceride und Polyvinylcaprolactam-Polyvinylacetat-Polyethylenglycol- Pfropfcopolymer,
d. 0.5 bis 25 % Gew. % Croscarmellose-Natrium
e. 0.1 bis 10 Gew. % kolloidales Siliziumdioxid
f. 0.1 bis 10 Gew. % Magnesiumstearat
g. 0.1 bis 10 Gew. % Weichmacher.

14. Ein Verfahren zur Herstellung einer pharmazeutischen Formulierung nach Anspruch 12, umfassend die Schritte
a. Vermischen von Flurbiprofen und Diacerein, Weichmacher und Polyvinylcaprolactam-Polyvinylacetat-Polyethylenglycol-Pfropfcopolymer oder Polyvinylalkohol-Polyethylenglycol-Copolymer, Schmelzen dieser Mischung und Hindurchführen durch einen Extruder oder ein Sieb,
b. Zugeben von zuerst Croscarmellose-Natrium und kolloidalem Siliziumdioxid und dann Magnesiumstearat zu den erhaltenen Granula und Vermischen derselben,
c. Durchführen eines Kompressionsschritts auf diese Pulvermischung in einer Tablettierungsmaschine, oder Einfüllen dieser Pulvermischung in Kapseln.

15. Ein Verfahren zur Herstellung einer pharmazeutischen Formulierung nach irgendeinem der vorstehenden Ansprüche, umfassend die Schritte
a. Vermischen von Flurbiprofen und Diacerein, Weichmacher, Stearyl-Macrogol-Glyceriden und Polyvinylcaprolactam-Polyvinylacetat-Polyethylenglycol- Pfropfcopolymer oder Polyvinylalkohol-Polyethylenglycol-Copolymer, Schmelzen dieser Mischung und Hindurchführen durch einen Extruder oder ein Sieb,
b. Zugeben von zuerst Croscarmellose-Natrium und kolloidalem Siliziumdioxid und dann Magnesiumstearat zu den erhaltenen Granula und Vermischen derselben,
c. Durchführen eines Kompressionsschritts auf diese Pulvermischung in einer Tablettierungsmaschine, oder Einfüllen dieser Pulvermischung in Kapseln.

## Revendications

1. Une formulation pharmaceutique, **caractérisée par** le fait de comprendre du flurbiprofène ou un sel pharmaceutiquement acceptable de flurbiprofène et de la diacéréine ou un sel pharmaceutiquement acceptable de diacéréine et un copolymère greffé caprolactame de polyvinyle - acétate de polyvinyle - polyéthylène glycol.

2. La formulation pharmaceutique selon la revendication 1, dans laquelle ladite formulation est obtenue au moyen d'un procédé de fusion à chaud n'impliquant aucun solvant liquide durant la phase de granulation.

3. La formulation pharmaceutique selon l'une quelconque des revendications précédentes, dans laquelle la proportion de flurbiprofène et de diacéréine par rapport au copolymère greffé caprolactame de polyvinyle - acétate de polyvinyle - polyéthylène glycol se situe dans la plage allant de 0,1 à 10, de préférence de 0,15 à 5 et plus préférentiellement de 0,2 à 2.

4. La formulation pharmaceutique selon l'une quelconque des revendications précédentes, comprenant en outre au moins un ou plusieurs excipients.

5. La formulation pharmaceutique selon l'une quelconque des revendications précédentes, dans laquelle ledit excipient est choisi dans le groupe constitué par au moins un ou un mélange de diluants, liant, désintégrants, agents de glissement, lubrifiants et plastifiants.

6. La formulation pharmaceutique selon l'une quelconque des revendications précédentes, dans laquelle la taille moyenne de particules (d₅₀) des granulés obtenus au moyen du procédé de fusion à chaud se situe dans la plage allant de 100 à 1500 µm, de préférence de 150 à 1000 µm et plus préférablement de 250 à 800 µm.

7. La formulation pharmaceutique selon l'une quelconque des revendications précédentes, comprenant en outre au moins un polymère dans lequel ledit polymère est choisi dans le groupe constitué par au moins un ou un mélange de copolymère alcool polyvinylique - polyéthylène glycol, copolymère bloc polyoxyéthylène - polyoxypropylène, glycéride de stéaryl-macrogole, polyéthylène gylcol, povidone, méthacrylate cationique, copovidone, dérivés de copolymère d'acide méthacrylique, phtalate d'acétate de cellulose, monoglycéride acétylé, tartrate de dibutyle, phtalate de diéthyle, phtalate de diméthye, glycérine, propylène glycol, acide stéarique, triacétine, citrate de triacétine et tripropionine.

8. La formulation pharmaceutique selon l'une quelconque des revendications précédentes, dans laquelle ledit désintégrant est choisi dans le groupe constitué par au moins un ou un mélange de croscarmellose de sodium et de glycolate d'amidon sodique.

9. La formulation pharmaceutique selon l'une quelconque des revendications précédentes, dans laquelle ledit agent de glissement est du dioxyde de silicium colloïdal.

10. La formulation pharmaceutique selon l'une quelconque des revendications précédentes, dans laquelle lesdits lubrifiants sont choisis dans le groupe constitué par au moins un ou un mélange polyéthylène glycol et de stéarate de magnésium.

11. La formulation pharmaceutique selon l'une quelconque des revendications précédentes, dans laquelle ledit plastifiant est choisi dans le groupe constitué par au moins un ou un mélange d'huile de ricin, glycérine, citrates d'ester (citrate d'acétyl-tri-n-butyle, citrate d'acétyl-triéthyle, citrate de tri-n-butyle, citrate de triéthyle), sébaçate de dibutyle, triacétine et phtalate de diéthyle.

12. La formulation pharmaceutique selon la revendication 7, constituée de :
a. 15 à 70% en poids de flurbiprofène ou d'un sel pharmaceutiquement acceptable de celui-ci,
b. 5 à 70% en poids de diacéréine ou d'un s el pharmaceutiquement acceptable de celle-ci,
c. 5 à 80% en poids de copolymère greffé caprolactame de polyvinyle - acétate de polyvinyle - polyéthylène glycol ou de copolymère alcool polyvinylique - polyéthylène glycol,
d. 0,5 à 25% en poids de croscarmellose de sodium,
e. 0,1 à 10% en poids de dioxyde de silicium colloïdal,
f. 0,1 à 10% en poids de stéarate de magnésium,
g. 0,1 à 10% en poids de plastifiant.

13. La formulation pharmaceutique selon l'une quelconque des revendications précédentes, constituée de :
a. 15 à 70% en poids de flurbiprofène ou d'un sel pharmaceutiquement acceptable de celui-ci,
b. 5 à 70% en poids de diacéréine ou d'un sel pharmaceutiquement acceptable de celle-ci,
c. 0,5 à 80% en poids de glycérides de stéaryl-macrogole et d'un copolymère greffé caprolactame de polyvinyle - acétate de polyvinyle - polyéthylène glycol,
d. 0,5 à 25% en poids de croscarmellose de sodium,
e. 0,1 à 10% en poids de dioxyde de silicium colloïdal,
f. 0,1 à 10% en poids de stéarate de magnésium,
g. 0,1 à 10% en poids de plastifiant.

14. Un procédé de préparation d'une formulation pharmaceutique selon la revendication 12, comprenant les étapes consistant à :
a. mélanger le flurbiprofène et la diacéréine, le plastifiant et le copolymère greffé caprolactame de polyvinyle - acétate de polyvinyle - polyéthylène glycol ou le copolymère alcool polyvinyle - polyéthylène glycol, faire fondre ce mélange et le faire passer à travers une extrudeuse ou un tamis,
b. ajouter d'abord le croscarmellose de sodium et le dioxyde de silicium colloïdal, et ensuite le stéarate de magnésium aux granulés obtenus et mélanger l'ensemble,
c. mettre en oeuvre une étape de compression sur ce mélange poudreux dans une machine à comprimés ou introduire ce mélange poudreux dans des gélules.

15. Un procédé de préparation d'une formulation pharmaceutique selon l'une quelconque des revendications précédentes, comprenant les étapes consistant à :
a. mélanger le flurbiprofène et la diacéréine, le plastifiant, les glycérides de stéaryle macrogol et le copolymère greffé caprolactame de polyvinyle - acétate de polyvinyle - polyéthylène glycol ou le copolymère alcool polyvinylique polyéthylène glycol, faire fondre ce mélange et le faire passer à travers une extrudeuse ou un tamis,
b. ajouter d'abord le croscarmellose de sodium et le dioxyde de silicium colloïdal puis le stéarate de magnésium aux granulés obtenus, puis mélanger l'ensemble,
c. mettre en oeuvre une étape de compression sur ce mélange poudreux dans une machine à comprimés ou introduire ce mélange poudreux dans des gélules.
